Europäisches Patentamt

European Patent Office

Office européen des brevets

(11) Veröffentlichungsnummer: **0 358 105 B1**

(12) **EUROPÄISCHE PATENTSCHRIFT**

(45) Veröffentlichungstag der Patentschrift: **02.03.94**

(21) Anmeldenummer: **89116065.7**

(22) Anmeldetag: **31.08.89**

(51) Int. Cl.5: **A61J 3/10, A61K 9/20, B30B 11/00**

(54) **Verfahren und Vorrichtung zur kontinuierlichen Herstellung von festen pharmazeutischen Formen.**

(30) Priorität: **07.09.88 DE 3830353**

(43) Veröffentlichungstag der Anmeldung:
**14.03.90 Patentblatt 90/11**

(45) Bekanntmachung des Hinweises auf die
Patenterteilung:
**02.03.94 Patentblatt 94/09**

(84) Benannte Vertragsstaaten:
**AT BE CH DE FR GB IT LI NL SE**

(56) Entgegenhaltungen:
**EP-A- 0 240 904          EP-A- 0 240 906
CH-A- 530 944          DE-A- 1 766 546
DE-C- 59 713          DE-C- 612 500
FR-A- 879 331          GB-A- 128 409
GB-A- 640 440          US-A- 2 829 756**

(73) Patentinhaber: **BASF Aktiengesellschaft
Carl-Bosch-Strasse 38
D-67063 Ludwigshafen(DE)**

(72) Erfinder: **Klimesch, Roger, Dr.
Georg-Froeba-Strasse 43
D-6146 Alsbach-Haehnlein 2(DE)**
Erfinder: **Bleckmann, Gerhard
Giselherstrasse 9
D-6840 Lampertheim 5(DE)**
Erfinder: **Farwerck, Karl-Peter
Enzingerstrasse 32
D-6520 Worms 21(DE)**
Erfinder: **Schlemmer, Lothar
Duisbergstrasse 1 a
D-6701 Maxdorf(DE)**
Erfinder: **Sanner, Axel, Dr.
Lorscher Ring 2 c
D-6710 Frankenthal(DE)**

**Beschreibung**

Die Erfindung betrifft ein kontinuierliches Verfahren zur Herstellung von festen pharmazeutischen Formen durch Extrusion einer wirkstoffhaltigen Polymerschmelze und Verformen des noch plastischen Stranges zwischen einem Band und einer Walze oder zwei Bändern sowie die Vorrichtung dazu.

Es ist bekannt, daß man pharmazeutische Wirkstoffe enthaltende Polymerschmelzen extrudieren und durch Spritzguß oder Kalandrieren verformen kann (EP-A-240 904 und 240 906). Das Spritzgußverfahren ist nicht vollkontinuierlich, sondern arbeitet mit sich im Takt wiederholenden Arbeitsgängen, die sich wegen der erforderlichen Kühlzeiten nicht in dem für eine Großserienfertigung erforderlichen Maß beschleunigen lassen. Auch beim Kalandrieren ist die Produktionsgeschwindigkeit begrenzt, weil die Walzen sich nur entlang einer Linie berühren, so daß die Kühlzeit nur bei langsam laufenden Walzen ausreicht, um den heißen, noch plastischen Strang soweit abzukühlen, daß die erhaltenen Formlinge dimensionsstabil sind.

US 2 829 756 beschreibt eine komplizierte Maschine, bei der ein extrudierter plastischer Strang durch mitlaufende formgebende Stanzen zu länglichen, zylindrischen Formen zerschnitten wird.

Der Erfindung lag daher die Aufgabe zugrunde, ein Verfahren zur kontinuierlichen Herstellung von festen pharmazeutischen Formen zu entwickeln, das zum einen eine Großproduktion und zum anderen auch die Verarbeitung von nur langsam aushärtenden Schmelzen erlaubt.

Die Lösung dieser Aufgabe besteht in den Verfahren und Vorrichtungen nach den Ansprüchen.

Wenn auch Fälle denkbar sind, in denen ein Vormischen zweckmäßig ist, so daß ein einfacher Extruder genügt, so ist es doch in der Regel wesentlich vorteilhafter, wenn der Extruder in üblicher Weise als ein- oder mehrwelliger Mischextruder gestaltet ist, so daß ein Vormischen nicht erforderlich ist. Der Mischextruder (1) kann mehrere Einfüllstutzen (2), gegebenenfalls für die getrennte Zugabe von festen oder flüssigen Mischungsbestandteilen, sowie einen Rohrstutzen zur Inertbegasung (in der Regel Stickstoff) und/oder Entgasung besitzen. Zur Erhöhung des Durchsatzes kann der Extruder mehr als eine Düse (3) haben.

Um einen sicheren Transport des extrudierten Stranges zu gewährleisten und sein Abreißen hinter der Düse zu vermeiden, extrudiert man zweckmäßig schräg nach unten. Der jeweils zweckmäßigste Winkel hängt von den Produkteigenschaften und der Fahrweise (z.B. Extrusionstemperatur und -geschwindigkeit) ab.

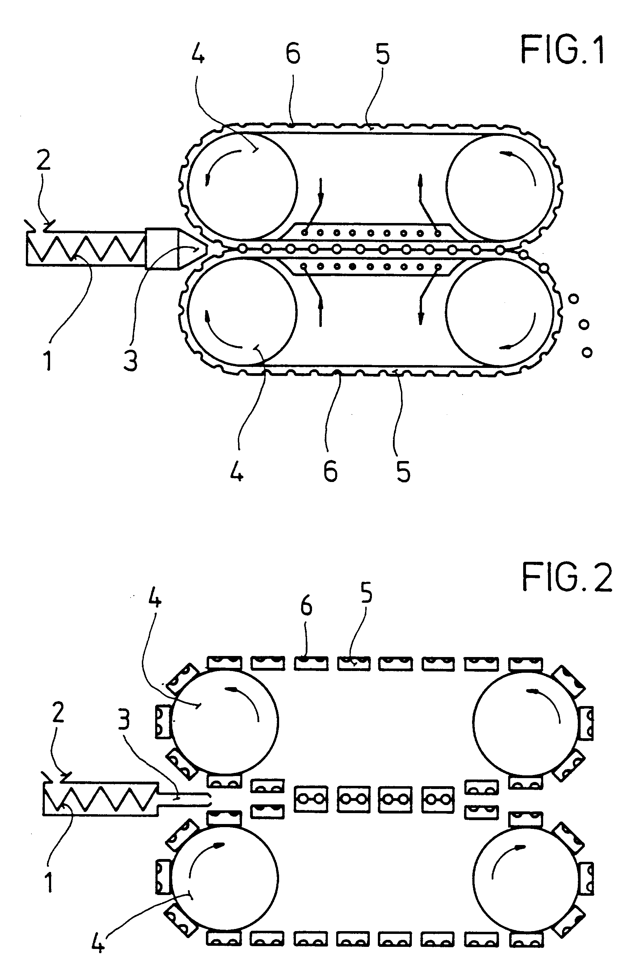

Die Formgebung schließt sich unmittelbar an den Extrusionsvorgang an. Man führt den noch plastischen extrudierten Strang, gegebenenfalls mit Hilfe einer geeigneten Führungsrinne (8), durch die in den Figuren 1 bis 6 beschriebenen Vorrichtungen zur Formgebung.

Im allgemeinen ist es sinnvoll, die formgebenden Teile (Walze und Band bzw. Doppelband) auf 10 bis 20°C zu kühlen. Tiefere Temperaturen sind ohne erheblichen Aufwand unzweckmäßig wegen der zu erwartenden Kondensatbildung. Die formgebenden Teile sind daher vorzugsweise mit den üblichen Kühlvorrichtungen zum Kühlen mit einer Kühlflüssigkeit versehen. In manchen Fällen genügt auch die natürliche Luftkühlung. Es kann auch zweckmäßig sein, die formgebenden Teile zu beheizen.

Falls der Extruder mehr als eine Düse hat, gehört bzw. gehören zu jeder Düse eine oder mehrere Reihen umlaufender formgebender Vertiefungen in der Walze und/oder im Band bzw. (beim Doppelband) in einem oder beiden Bändern.

Im Fall der elastischen Bänder nach Anspruch 2 (Fig. 1) sind in den Mantelflächen der Bänder jeweils einander gegenüberliegende formgebende Vertiefungen angebracht, die paarweise die Tablettenform bestimmen. Zweckmäßig enthält die Vorrichtung eine übliche Steuer- und Regeleinrichtung, die dafür sorgt, daß die beiden Formhälften jeweils exakt zusammentreffen. Die Bänder bestehen aus einem gefüllten Elastomer, beispielsweise mit Aluminiumpulver oder -Schuppen gefülltem Polypropylen, Acrylnitril-Butadien-Styrol-Copolymeren, Polyamid, Polycarbonat oder deren Mischungen, wobei der Füllstoff die Wärmeleitfähigkeit verbessert, und die Bandstärke ist etwas größer als die Tiefe der Formhälfte.

Metallgliederbänder (Fig. 2) können aus verschiedenen Metallen wie Messing, Bronze, Kupfer, vorzugsweise aus korrosions- und abrasionsfestem Stahl bestehen. Die Bänder sind unterteilt in Segmente (Glieder), die formgebende Vertiefungen enthalten. Pro Segment können sowohl in der Längsrichtung wie auch nebeneinander mehrere formgebende Vertiefungen eingraviert sein.

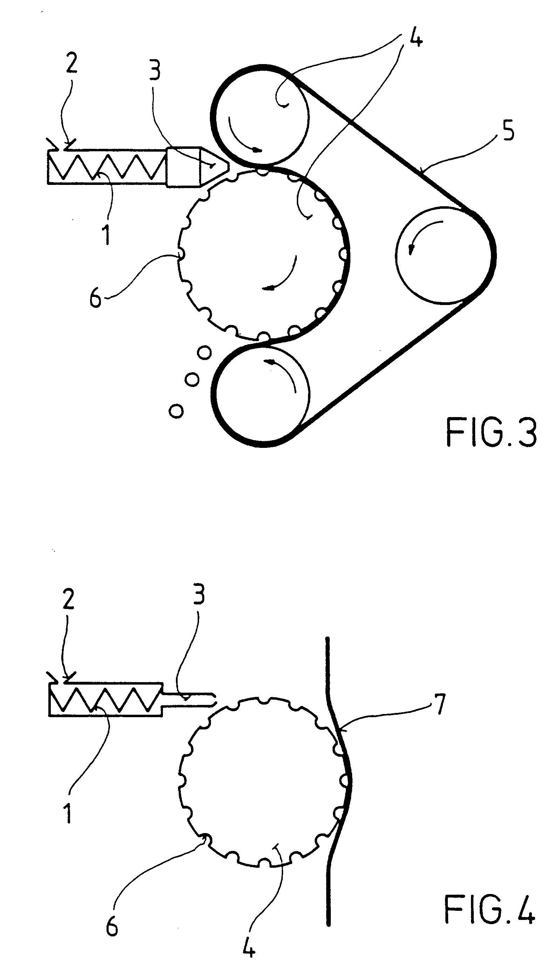

Im Fall glatter Bänder in Kombination mit gravierten Walzen nach Anspruch 4 (Fig. 3) können die Bänder sowohl aus Elastomeren wie aus Metall bestehen, bevorzugt werden dünne Stahlbänder.

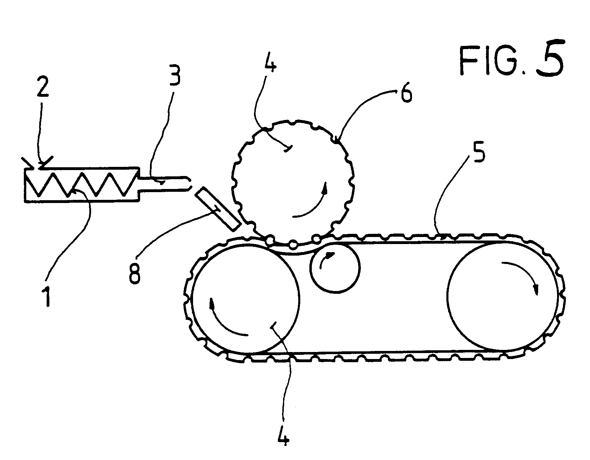

Das glatte Band kann auch durch eine feststehende glatte, ebene oder vorzugsweise in Anpassung an die Walze konkav gewölbte Wand ersetzt sein (Anspruch 4; Fig. 4).

Schließlich können Walze (4) und Band (5) mit miteinander paarweise korrespondierenden formgebenden Vertiefungen (6) versehen sein (Anspruch 5; Fig. 5).

Aufgrund der längeren Kontaktzeit zwischen den Bändern bzw. dem Band und der Walze ist die Kühlzeit im Vergleich mit dem in EP-A-240 906 beschriebenen Walzenpaar, das sich nur an einer Linie

2

berührt, wesentlich verlängert. Dadurch kann einerseits der Durchsatz erhöht werden, indem man die Umdrehungsgeschwindigkeit im Vergleich zum Walzenpaar steigert, andererseits können auch pharmazeutische Mischungen verarbeitet werden, die nur sehr langsam sich verfestigen. Am längsten ist die Abkühlzeit bei Anwendung zweier Bänder (vgl. Fig. 1 und 2).

Bei Verwendung einer gravierten Walze in Kombination mit einem glatten Band ist sowohl eine Anordnung nach Fig. 3 wie nach dem Prinzip der Fig. 5 möglich. Bei der Anordnung nach Fig. 3 wird zweckmäßig nur die Walze gekühlt, während bei der Anordnung nach dem Prinzip der Fig. 5 Walze und Band gekühlt werden können, es ist aber auch in besonderen Fällen möglich, das Band zu kühlen und die Walze zu beheizen. Bei beiden Anordnungen kann der Umschlingungswinkel (das vom Band umschlossene Walzensegment) selbstverständlich größer oder kleiner als in der Zeichnung sein.

Die Elemente der Vorrichtung sind jeweils so anzuordnen, daß der Formling am Ende der Kühlstrecke nach unten herausfallen kann. Es empfiehlt sich jedoch sicherheitshalber, zusätzlich eine Abstreifwalze vorzusehen, die für eine zuverlässige Entformung sorgt, ohne die Formlinge zu beschädigen. Die Abstreifwalze besitzt daher zweckmäßig weiche Borsten. Gleichzeitig reinigt sie die Form.

Extrudierbare pharmazeutische Mischungen sind Mischungen mindestens eines pharmazeutischen Wirkstoffes mit mindestens einem für die Herstellung pharmazeutischer Tabletten üblichen Hilfsstoff, die durch Schmelzen oder Erweichen mindestens einer Komponente teigig und daher extrudierbar sind. Das sind insbesondere Mischungen, die pharmakologisch akzeptable Polymere enthalten (wobei die Glastemperatur der Mischung unter der Zersetzungstemperatur aller Mischungskomponenten liegt), beispielsweise Polyvinylpyrrolidon (PVP), Copolymerisate von N-Vinylpyrrolidon (NVP) und Vinylacetat, Copolymerisate von Vinylacetat und Crotonsäure, teilverseiftes Polyvinylacetat, Polyvinylalkohol, Ethylen/Vinylacetat-Copolymerisate, Polyhydroxyethylmethacrylat, Copolymerisate von Methylmethacrylat und Acrylsäure, Celluloseester, Celluloseether, Polyethylenglykol, Polyethylen. Die K-Werte (nach H. Fikentscher, Cellulose-Chemie 13 (1932), Seiten 58 bis 64 und 71 und 74) der Polymeren liegen im Bereich von 10 bis 100, vorzugsweise 12 bis 70, insbesondere 12 bis 35, für PVP bei 12 bis 70, vorzugsweise bei 12 bis 35, insbesondere bei 12 bis 17.

Das polymere Bindemittel muß in der Gesamtmischung aller Komponenten im Bereich von 50 bis 180, vorzugsweise 60 bis 130°C erweichen oder schmelzen, so daß die Masse extrudierbar ist. Die Glasübergangstemperatur der Mischung muß also auf jeden Fall unter 180, vorzugsweise unter 130°C liegen. Erforderlichenfalls wird sie durch übliche pharmakologisch akzeptable weichmachende Hilfsstoffe wie langkettige Alkohole, Ethylenglykol, Propylenglykol, Trimethylolpropan, Triethylenglykol, Butandiole, Pentanole, Hexanole, Polyethylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester) oder Fettsäureester herabgesetzt.

Bevorzugt werden NVP-Polymerisate, die in Mischung mit dem Wirkstoff und ggf. üblichen galenischen Hilfsstoffen mit oder vorzugsweise ohne weichmachende Zusätze im gewünschten Temperaturbereich schmelzen oder erweichen. Das Schmelzen oder Erweichen unterhalb einer bestimmten Temperatur ist gegebenenfalls erforderlich im Hinblick auf eine mögliche thermische und/oder oxidative Schädigung nicht nur des Wirkstoffs, sondern auch des NVP-Polymerisates. Dieses könnte beim Extrudieren vergilben, weshalb die Extrusion von NVP-Polymerisaten bisher nicht üblich ist. Die Gefahr ist jedoch gering bei Extrusionstemperaturen unter 180°C, vor allem unter 130°C, wenn das Polymerisat nicht in wäßriger Lösung mit Wasserstoffperoxid als Starter hergestellt wurde, sondern in einem organischen Lösungsmittel, oder aber in Wasser mit einem organischen Peroxid als Starter, etwa nach dem Verfahren gemäß der EP-A-273 238 oder nach dem Verfahren von US 4 520 179 und 4 520 180.

Falls der K-Wert über 17, insbesondere über 30 oder gar 40 liegt und keine stark weichmachende Komponente zugegen ist, kommen als NVP-Polymerisate nur solche mit einer Glastemperatur Tg unter 120, vorzugsweise unter 100°C in Betracht, oder das NVP-Polymerisat (einschließlich Homopolymer) darf nicht in Wasser mit $H_2O_2$ als Starter hergestellt worden sein. Dabei würden Polymer-Endgruppen entstehen, die bei höherer Temperatur zur Vergilbung führen.

Das NVP-Polymerisat kann über die Art und Menge an Comonomeren je nach Anwendungszweck so stark oder so schwach hydrophil eingestellt werden, daß sich die daraus hergestellten Tabletten im Mund (Buccaltablette) oder im Magen oder auch erst im Darm (rasch oder verzögert) auflösen oder so quellen, daß sie den Wirkstoff freigeben. Sie sind dann ausreichend quellbar, wenn sie bei Lagerung bei 90 % relativer Luftfeuchtigkeit mehr als 10 Gew.% Wasser aufnehmen. Falls es bei carboxylgruppenhaltigen Bindemitteln erwünscht ist, daß sie erst im alkalischen Milieu des Darms den Wirkstoff freigeben, gilt die obige Angabe der Wasseraufnahme nur für die neutralisierte Form (Salzform) des Polymeren (in der die Protonen der Carboxylgruppen ganz oder teilweise durch Ammonium-, Natrium- oder Kaliumionen ersetzt sind).

Als Comonomer zum NVP kommen in Betracht: ungesättigte Carbonsäuren, z.B. Methacrylsäure, Crotonsäure, Maleinsäure, Itaconsäure, sowie deren Ester mit Alkoholen mit 1 bis 12, vorzugsweise 1 bis 8 Kohlenstoffatomen, ferner Hydroxyethyl- oder Hydroxypropylacrylat und -methacrylat, (Meth)acrylamid, die Anhydride und Halbester der Maleinsäure und Itaconsäure (wobei der Halbester vorzugsweise erst nach der Polymerisation gebildet wird), N-Vinylcaprolactam und Vinylpropionat. Bevorzugte Comonomere sind Acrylsäure und insbesondere Vinylacetat. Es werden daher NVP-Polymerisate bevorzugt, die entweder nur NVP oder Vinylacetat als einziges Comonomeres einpolymerisiert enthalten. Vinylacetat und Vinylpropionat können nach der Polymerisation ganz oder teilweise verseift sein.

Übliche galenische Hilfsstoffe, deren Gesamtmenge bis zu 100 Gew.%, bezogen auf das Polymerisat, betragen kann, sind z.B. Streckmittel wie Silikate oder Kieselerde, Stearinsäure oder deren Salze mit z.B. Magnesium oder Kalzium, Methylcellulose, Natrium-Carboxymethylcellulose, Talkum, Saccharose, Lactose, Getreide- oder Maisstärke, Kartoffelmehl, Polyvinylalkohol, ferner Netz-, Konservierungs-, Spreng-, Adsorptionsmittel, Farbstoffe, Geschmacksstoffe (vgl. z.B. H. Sucker et al., Pharmazeutische Technologie, Thieme-Verlag, Stuttgart 1978).

Falls gewünscht, kann die erfindungsgemäß hergestellte Tablette auch mit einem üblichen Überzug zur Verbesserung des Aussehens und/oder des Geschmacks (Dragee, Filmtabletten) oder zwecks zusätzlicher Verzögerung der Wirkstofffreigabe versehen werden. Für oral einzunehmende Tabletten mit verzögerter Wirkstofffreisetzung kann es günstig sein, wenn man die Tablette nach einer der bekannten Techniken in geschlossenzellig poröser Form herstellt, damit sie im Magen aufschwimmt und dadurch länger dort verweilt. Ferner können nach dem erfindungsgemäßen Verfahren sehr kleine Tabletten hergestellt werden, die mit Vorteil anstelle herkömmlicher Granulate in Kapseln abgefüllt werden. Der Begriff "Tablette" im Sinne der Erfindung ist weder an eine bestimmte Form noch an die perorale Anwendung gebunden. Er schließt vielmehr auch (nicht bei Körpertemperatur schmelzende) Zäpfchen zur rektalen Anwendung ein.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.% liegen. Auch Wirkstoff-Kombinationen können eingesetzt werden. Wirkstoffe im Sinne der Erfindung sind auch Vitamine.

Das erfindungsgemäße Verfahren ist beispielsweise zur Verarbeitung folgender Wirkstoffe geeignet:
Betamethason, Thioctsäure, Sotalol, Salbutamol, Norfenefrin, Silymarin, Dihydergotamin, Buflomedil, Etofibrat, Indometacin, Oxazepam, $\beta$-Acetyldigoxin, Piroxicam, Haloperidol, Isosorbidmononitrat, Amitriptylin, Diclofenac, Nifedipin, Verapamil, Pyritinol, Nitrendipin, Doxycyclin, Bromhexin, Methylprednisolon, Clonidin, Fenofibrat, Allopurinol, Pirenzepin, Levothyroxin, Tamoxifen, Metildigoxin, o-($\beta$-Hydroxyethyl)-rutosid, Propicillin, Aciclovirmononitrat, Paracetamol, Naftidrofuryl, Pentoxifyllin, Propafenon, Acebutolol, L-Thyroxin, Tramadol, Bromocriptin, Loperamid, Ketotifen, Fenoterol, Ca-Dobelisat, Propranolol, Minocyclin, Nicergolin, Ambroxol, Metoprolol, $\beta$-Sitosterin, Enalaprilhydrogenmaleat, Bezafibrat, ISDN, Gallopamil, Xantinolnicotinat, Digitoxin, Flunitrazepan, Bencyclan, Dexapanthenol, Pindolol, Lorazepam, Diltiazem, Piracetam, Phenoxymethylpenicillin, Furosemid, Bromazepam, Flunarizin, Erythromycin, Metoclopramid, Acemetacin, Ranitidin, Biperiden, Metamizol, Doxepin, Dikalium-Chlorazepat, Tetrazepam, Estramustinphosphat, Terbutalin, Captopril, Maprotilin, Prazosin, Atenolol, Glibenclamid, Cefaclor, Etilefrin, Cimetidin, Theophyllin, Hydromorphon, Ibuprofen, Primidon, Clobazam, Oxaceprol, Medroxyprogesteron, Flecainid, Mg-Pyridoxal-5-phosphatglutaminat, Hymechromon, Etofyllinclofibrat, Vincamin, Cinnarizin, Diazepam, Ketoprofen, Flupentixol, Molsidomin, Glibornurid, Dimetinden, Melperon, Soquinolol, Dihydrocodein, Clomethiazol, Clemastin, Glisoxepid, Kallidinogenase, Oxyfedrin, Baclofen, Carboxymethylcystein, Thioridacin, Betahistin, L-Tryptophan, Myrtol, Bromelaine, Prenylamin, Salazosulfapyridin, Astemizol, Sulpirid, Benzerazid, Dibenzepin, Acetylsalicylsäure, Miconazol, Nystatin, Ketoconazol, Na-Picosulfat, Colestyramin, Gemfibrocil, Rifampicin, Fluorcortolon, Mexiletin, Amoxicillin, Terfenadrin, Mucopolysaccharidpolyschwefelsäureester, Triazolam, Mianserin, Tiaprofensäure, Ameziniummetilsulfat, Mefloquin, Probucol, Chinidin, Carbamazepin, Mg-L-aspartat, Penbutolol, Piretanid, Amitriptylin, Cyproteron, Na-Valproinat, Mebeverin, Bisacodyl, 5-Amino-Salicylsäure, Dihydralazin, Magaldrat, Phenprocoumon, Amantadin, Naproxen, Carteolol, Famotidin, Methyldopa, Auranofin, Estriol, Nadolol, Levomepromazin, Doxorubicin, Medofenoxat, Azathioprin, Flutamid, Norfloxacin, Fendilin, Prajmaliumbitartrat, Aescin.

Besonders bevorzugt werden feste Lösungen folgender Wirkstoffe:
Acetaminophen (= Paracetamol), Acetohexamid, Acetyldigoxin, Acetylsalicylsäure, Acromycin, Anipamil, Benzocain, $\beta$-Carotin, Chloramphenicol, Chlordiazepoxid, Chlormadinonacetat, Chlorothiazid, Cinnarizin, Clonazepam, Codein, Dexamethason, Diazepam, Dicumarol, Digitoxin, Digoxin, Dihydroergotamin, Drotaverin, Flunitrazepam, Furosemid, Gramicidin, Griseofulvin, Hexobarbital, Hydrochlorothiazid, Hydrocortison, Hydroflumethiazid, Indomethazin, Ketoprofen, Lonetil, Medazepam, Mefrusid, Methandrostenolon, Methylprednisolon, Methylsulfadiazin (= Sulfaperin), Nalidixinsäure, Nifedipin, Nitrazepam, Nitrofurantoin, Nystatin, Östradiol, Papaverin, Phenacetin, Phenobarbital, Phenylbutazon, Phenytoin, Prednison, Reserpin, Spironolacton, Streptomycin, Sulfadimidin (= Sulfamethazin), Sulfamethizol, Sulfamethoxazol, Sulfamethoxydiazin (= Sulfameter), Sulfaperin, Sulfathiazol, Sulfisoxazol, Testosteron, Tolazamid, Tolbutamid, Trimethoprim, Tyrothricin.

Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymeren vor.

Die Bildung fester Lösungen der genannten Wirkstoffe in NVP-Polymerisaten war nicht vorherzusehen und ist umso überraschender, als viele in Wasser schwerlösliche Wirkstoffe in anderen Polymeren keine festen Lösungen (mit molekulardisperser Verteilung) bilden, sondern sich in Form fester Teilchen in das jeweilige Polymere einlagern, die elektronenmikroskopisch zu erkennen sind. Im Falle kristalliner Wirkstoffe zeigen sie auch ein Debye-Scherrer-Diagramm, im Gegensatz zu den festen Lösungen.

Die in den Beispielen genannten Teile und Prozente beziehen sich auf das Gewicht.

Beispiel 1 bis 32: Doppelgliederband gem. Fig. 2

Beispiel 1

45 Teile eines Copolymerisats vom K-Wert 30 aus 60 Gew.% N-Vinylpyrrolidon und 40 Gew.% Vinylacetat, 5 Teile Stearylalkohol und 50 Teile Theophyllin wurden in einem Doppelschneckenextruder vermischt und extrudiert. Die Temperatur des sechs Schüsse enthaltenden Extrudermantels betrug 30, 60, 60, 60, 80 und 100°C; die Düse wurde auf 100°C aufgeheizt. Der hierbei erhaltene Strang wurde direkt mit einem Doppelgliederband, das auf 15°C gekühlt wurde, zu Oblongtabletten verpreßt. Es entstanden starre Tabletten.

Die so erhaltenen Tabletten waren gegen mechanische Einflüsse stabil und zeigten keinen Abrieb beim Transport und Verpacken.

Beispiel 2

50 Teile des Copolymerisats von Beispiel 1 und 50 Teile Theophyllin wurden in einem Doppelschneckenextruder vermischt und extrudiert. Abweichend von Beispiel 1 wurde die Temperatur der Schüsse auf 30, 60, 60, 60, 90 und 120°C eingestellt. Die Düse hatte ebenfalls eine Temperatur von 120°C. Der erhaltene Strang wurde analog zu Beispiel 1 zu Oblongtabletten verpreßt. Die Temperatur des Doppelgliederbandes betrug 15°C. Auch diese analog Beispiel 1 erhaltenen Tabletten waren gegen mechanische Einflüsse stabil.

Beispiel 3

47,5 Teile eines Copolymerisats vom K-Wert 30 aus 60 Gew.% N-Vinylpyrrolidon und 40 Gew.% Vinylacetat, 2,5 Teile vernetztes PVP als Tablettensprengmittel und 50 Teile Theophyllin wurden in einem Doppelschneckenextruder vermischt und extrudiert. Die Temperatur der fünf Schüsse betrug jeweils 120°C, die Düse hatte eine Temperatur von 130°C.

Der noch plastische Strang wurde wie bei Beispiel 1 zu Oblongtabletten verpreßt (Temp. des Doppelgliederbandes: +15°C). Die Tabletten waren stabil gegen mechanische Einflüsse.

Beispiel 4

50 Teile eines Copolymerisats vom K-Wert 52 aus 30 Gew.% N-Vinylpyrrolidon und 70 Gew.% Vinylacetat und 50 Teile Theophyllin wurden in einem Doppelschneckenextruder gemischt und extrudiert. Die Temperatur der fünf Schüsse betrug 30, 60, 100, 100 und 120°C. Die Düse wurde ebenfalls auf 120°C aufgeheizt. Der noch plastische Strang wurde wie bei Beispiel 1 zu mechanisch stabilen Oblongtabletten verpreßt. (Temp. des Doppelgliederbandes +15°C.)

Beispiele 5 bis 8

Eine Mischung von 50 Gew.% eines N-Vinylpyrrolidon-Homopolymeren (PVP) von dem jeweils in der Tabelle angegebenen K-Wert und 50 Gew.% Theophyllin wurden in einem Einwellen-Extruder bei der jeweils in der Tabelle angegebenen Temperatur aufgeschmolzen, extrudiert und wie bei Beispiel 1 zu Tabletten verformt.

| Beispiel | K-Wert | T [°C] | | | | | Düse | Temp. des Doppelgliederbandes [°C] |
|---|---|---|---|---|---|---|---|---|
| | | 1. | 2. | 3. | 4. | 5. | | |
| | | Schuß | | | | | | |
| 5 | 12 | 115 | 125 | 135 | 135 | 135 | 145 | 10 |
| 6 | 17 | 125 | 125 | 135 | 145 | 145 | 155 | 10 |
| 7 | 25 | 145 | 155 | 165 | 175 | 175 | 175 | 15 |
| 8 | 30 | 150 | 160 | 160 | 170 | 180 | 180 | 15 |
| 8a | 60 | 150 | 160 | 160 | 170 | 180 | 180 | 15 |

Beispiel 9

40 Teile eines Copolymerisats aus 60 Gew.% N-Vinylpyrrolidon und 40 Gew.% Vinylacetat vom K-Wert 30, 10 Teile Polyhydroxyethylmethacrylat und 50 Teile Theophyllin wurden analog zu Beispiel 1 zu mechanisch stabilen Tabletten verarbeitet. Temperatur der Schüsse: 70, 80, 80, 80, 80°C. Düse: 90°C. Doppelgliederband +30°C.

Beispiel 10

50 Teile eines handelsüblichen, zu 80 % verseiften Polyvinylacetats und 50 Teile Theophyllin wurden analog zu Beispiel 1 verarbeitet. Die Temperatur der 5 Schüsse betrug 100, 100, 110, 120, 130°C. Düse: 150°C. Doppelgliederband: +32°C.

Beispiel 11

50 Teile Polyhydroxyethylmethacrylat vom K-Wert 30 und 50 Teile Theophyllin wurden analog Beispiel 1 verarbeitet. Temperatur der Schüsse: 120, 130, 150, 160, 160°C. Düse: 170°C. Doppelgliederband: +30°C.

Beispiel 12 bis 14

36 Teile eines Copolymerisates aus 60 Gew.% N-Vinylpyrrolidon und 40 Gew.% Vinylacetat vom K-Wert 30, 4 Teile Stearylalkohol, 40 Teile Theophyllin und 20 Teile
Beispiel 12) Stärke
Beispiel 13) Lactose
Beispiel 14) Saccharose
wurden in einem 6-schüssigen Dopppelschneckenextruder gemischt und analog Beispiel 1 zu Tabletten verformt. Die Temperatur der Schüsse betrug 90, 100, 110, 120, 120, 130°C, die der Düse 135°C. Doppelgliederband: +15°C.

Beispiele 15 bis 17

50 Teile des Copolymerisates der Beispiele 12 bis 14 und 50 Teile Verapamil wurden gemäß den Beispielen 12 bis 14 zu Tabletten geformt.
Analog den obigen Beispielen wurden die folgenden durchgeführt. Die Bedingungen der Verarbeitung sowie die Freisetzungsgeschwindigkeiten beim half-change-Test (vgl. R. Voigt, Lehrbuch der pharmazeutischen Technologie, 5. Aufl., Verl. Chemie, Weinheim; Deerfield Beach, Florida; Basel, 1984, S. 627, in Verbindung mit der Paddle-Methode nach USP 21) sind tabellarisch erfaßt. Zur Formgebung wurde ein temperierbares Doppelgliederband (Beispiele 18 bis 32), ein temperierbares Doppelband (Bsp. 33 bis 53) und eine gravierte Walze zusammen mit einem glatten Band (Bsp. 54 bis 85) verwendet.

Tabelle 1: Doppelbandglieder gem. Fig. 2

| Beisp. Nr. | Wirkstoff | Polymer | Hilfs-stoff | Gew.-Verhältn. Wirkst./Polymer/ Hilfsstoff | T1 | T2 [°C] |
|---|---|---|---|---|---|---|
| 18 | Pseudoephedrin 47,5 Diphenhydramin 2,5 | A | ./. | 50/50/0 | 60 | 80 |
| 19 | Propafenon | A | Stärke | 40/40/20 | 60 | 70 |
| 20 | Propafenon | A | StA | 60/35/5 | 80 | 90 |
| 21 | Propafenon | A | StA | 60/30/10 | 80 | 90 |
| 22 | Propafenon | A | StS | 60/35/5 | 70 | 90 |
| 23 | Propafenon | B | StA | 50/40/10 | 65 | 80 |
| 24 | Propafenon | A | MgSt | 60/35/5 | 60 | 70 |
| 25 | Propafenon | A | MgSt | 50/40/10 | 60 | 70 |
| 26 | Anipamil | A | MgSt | 50/40/10 | 30 | 30 |
| 27 | Vitamin B1 | B | ./. | 50/50/0 | 40 | 40 |
| 28 | Nicotinsäure | A | ./. | 50/50/0 | 60 | 70 |
| 29 | Biperiden | A | StA | 50/45/5 | 80 | 90 |
| 30 | Biperiden | A | ./. | 50/50/0 | 80 | 90 |
| 31 | Canthaxantin | B | ./. | 50/50/0 | 30 | 30 |
| 32 | Canthaxantin | A | ./. | 50/50/0 | 40 | 40 |

Tabelle 1 (Fortsetzung)

| Beisp. Nr. | T3 | T4 | T5 [°C] | T6 | Düse | Freisetzungs-geschwindig-keit | Temperatur des Doppelglieder-bandes [°C] |
|---|---|---|---|---|---|---|---|
| 18 | 100 | 120 | 120 | 120 | 120 | 100 % in 1 h | 16 |
| 19 | 90 | 110 | 110 | 110 | 110 | 100 % in 1 h | 16 |
| 20 | 100 | 120 | 140 | 140 | 140 | 100 % in 2 h | 15 |
| 21 | 100 | 120 | 130 | 130 | 140 | 52 % in 6 h | 15 |
| 22 | 100 | 110 | 115 | 115 | 115 | 42 % in 6 h | 15 |
| 23 | 95 | 110 | 110 | 110 | 110 | 100 % in 6 h | 15 |
| 24. | 80 | 80 | 95 | 100 | 100 | 95 % in 6 h | 10 |
| 25 | 80 | 80 | 95 | 100 | 100 | 80 % in 6 h | 10 |
| 26 | 40 | 40 | 60 | 60 | 60 | 100 % in 2 h | 10 |
| 27 | 50 | 60 | 80 | 80 | 80 | 100 % in 1 h | 10 |
| 28 | 80 | 95 | 95 | 100 | 100 | 100 % in 1 h | 10 |
| 29 | 100 | 120 | 120 | 130 | 135 | 100 % in 4 h | 16 |
| 30 | 110 | 120 | 140 | 140 | 140 | 100 % in 1 h | 16 |
| 31 | 40 | 40 | 60 | 60 | 60 | 100 % in 1 h | 20 |
| 32 | 55 | 60 | 60 | 80 | 80 | 100 % in 1 h | 20 |

Tabelle 2: Doppelband gem. Fig. 1

| Beisp. Nr. | Wirkstoff | Polymer | Hilfs- stoff | T1 | T2 | T3 | T4 | T5 | T6 | Düse | Temperatur des Doppelglieder- bandes [°C] |
|---|---|---|---|---|---|---|---|---|---|---|---|
| | | | | | | | [°C] | | | | |
| 33 | Indomethacin | A | | 50 | 60 | 70 | 80 | 80 | 80 | 80 | 10 |
| 34 | Indomethacin | B | | 60 | 80 | 100 | 120 | 120 | 120 | 120 | 10 |
| 35 | Anipamil | A | | 30 | 30 | 40 | 50 | 50 | 60 | 60 | 15 |
| 36 | Anipamil | B | | 30 | 30 | 40 | 50 | 50 | 60 | 60 | 15 |
| 37 | Benzocain | D | | 60 | 80 | 95 | 100 | 120 | 120 | 140 | 20 |
| 38 | Benzocain | D | | 60 | 80 | 95 | 100 | 120 | 130 | 140 | 20 |
| 39 | Benzocain | F | | 30 | 30 | 40 | 50 | 50 | 60 | 60 | 10 |
| 40 | Benzocain | B | | 60 | 80 | 100 | 120 | 120 | 120 | 120 | 10 |
| 41 | 5,5-Diphenhydramin | B | | 60 | 80 | 100 | 120 | 120 | 120 | 120 | 15 |
| 42 | Paracetamid | B | | 60 | 80 | 100 | 120 | 120 | 120 | 120 | 15 |
| 43 | Sulfathiazol | B | | 70 | 90 | 100 | 100 | 100 | 100 | 120 | 10 |
| 44 | Sulfathiazol | E | | 70 | 90 | 100 | 100 | 100 | 110 | 120 | 15 |
| 45 | Benzocain | A | | 30 | 30 | 40 | 50 | 60 | 70 | 70 | 10 |
| 46 | 5,5-Diphenhydramin | A | | 60 | 80 | 100 | 120 | 120 | 120 | 130 | 10 |
| 47 | Paracetamol | A | | 60 | 80 | 100 | 120 | 120 | 120 | 130 | 10 |
| 48 | Sulfathiazol | A | | 70 | 90 | 100 | 100 | 100 | 100 | 130 | 10 |
| 49 | Vitamin C | C | | 75 | 95 | 95 | 120 | 120 | 120 | 120 | 20 |
| 50 | Benzocain | E | | 60 | 70 | 80 | 120 | 130 | 130 | 130 | 15 |
| 51 | Benzocain | G | | 60 | 70 | 70 | 80 | 80 | 80 | 120 | 15 |
| 52 | Benzocain | H | | 50 | 60 | 60 | 60 | 80 | 90 | 110 | 10 |
| 53 | Benzocain | I | | 50 | 60 | 70 | 70 | 75 | 75 | 80 | 10 |

EP 0 358 105 B1

Tabelle 3: Gravierte Walze + glattes Band gem. Fig. 3

| Beisp. Nr. | Wirkstoff | Polymer | Hilfs- stoff | Gew.-Verhältnis Wirkst./Polymer/ Hilfsstoff | T1 | T2 | T3 | T4 [°C] | T5 | T6 | Düse | Temp. der Walze [°C] |
|---|---|---|---|---|---|---|---|---|---|---|---|---|
| 54 | Metoprolol | A | StA | 40/55/5 | 60 | 70 | 80 | 80 | 90 | 80 | 80 | 18 |
| 55 | Ranitidin | A | – | 46/54/o | 60 | 70 | 80 | 80 | 90 | 90 | 80 | 18 |
| 56 | Diclophenac | A | StA | 40/55/5 | 65 | 70 | 80 | 90 | 90 | 90 | 90 | 18 |
| 57 | Furosemid | A | StA | 30/60/10 | 65 | 75 | 80 | 90 | 100 | 100 | 100 | 18 |
| 58 | Nifedipin | A | StA | 20/70/10 | 60 | 70 | 80 | 80 | 80 | 80 | 80 | 18 |
| 59 | Gallopamil | A | StA | 40/54/6 | 50 | 60 | 70 | 80 | 80 | 70 | 70 | 16 |
| 60 | Gallopamil | A | StA | 40/48/12 | 50 | 60 | 70 | 80 | 80 | 70 | 70 | 16 |
| 61 | Gallopamil | A | StA | 40/42/18 | 50 | 60 | 70 | 80 | 80 | 70 | 70 | 16 |
| 62 | Gallopamil | A | StS | 40/54/6 | 50 | 60 | 70 | 80 | 80 | 70 | 70 | 16 |
| 63 | Gallopamil | A | StS | 40/48/12 | 50 | 60 | 70 | 80 | 80 | 70 | 70 | 16 |
| 64 | Gallopamil | A | StS | 40/42/18 | 50 | 60 | 70 | 80 | 80 | 70 | 70 | 16 |
| 65 | Anipamil | A | StA | 34/54,4/13,6 | 50 | 60 | 65 | 65 | 60 | 60 | 55 | 10 |
| 66 | Biperiden | A | StA | 6/89/5 | 45 | 55 | 60 | 65 | 65 | 65 | 60 | 15 |
| 67 | Biperiden | A | StA | 6/84/10 | 45 | 55 | 50 | 65 | 65 | 65 | 60 | 15 |
| 68 | Biperiden | A | StA | 6/79/15 | 45 | 55 | 60 | 65 | 65 | 65 | 60 | 15 |
| 69 | Biperiden | A | StA | 6/74/20 | 50 | 50 | 60 | 60 | 50 | 50 | 50 | 10 |
| 70 | Biperiden | A | StA | 6/69/25 | 40 | 50 | 55 | 60 | 60 | 50 | 50 | 10 |
| 71 | Biperiden | A | StA | 6/64/30 | 40 | 50 | 55 | 60 | 60 | 50 | 50 | 10 |
| 72 | Biperiden | A | StA | 6/59/35 | 40 | 50 | 55 | 60 | 60 | 50 | 50 | 10 |
| 73 | Bezafibrat | A | – | 61,5/38,5/0 | 60 | 70 | 80 | 80 | 80 | 80 | 80 | 15 |
| 74 | Bezafibrat | A | StA | 61,5/34/4,5 | 60 | 70 | 80 | 80 | 80 | 70 | 70 | 15 |
| 75 | Bezafibrat | A | StA | 61,5/29,5/9,0 | 40 | 45 | 50 | 50 | 50 | 50 | 50 | 15 |

EP 0 358 105 B1

Tabelle 3: Fortsetzung

| Beisp. Nr. | Wirkstoff | Polymer | Hilfs-stoff | Gew.-Verhältnis Wirkst./Polymer/Hilfsstoff | T1 | T2 | T3 | T4 | T5 | T6 | Düse | Temp. der Walze |
|---|---|---|---|---|---|---|---|---|---|---|---|---|
| 76 | Metoprolol | A | Stärke | 40/45/15 | 60 | 70 | 80 | 80 | 80 | 80 | 80 | 15 |
| 77 | Metoprolol | A | Stärke | 40/35/25 | 55 | 60 | 65 | 70 | 70 | 70 | 70 | 16 |
| 78 | Anipamil | A | Lactose | 32/43/25 | 55 | 60 | 70 | 80 | 70 | 70 | 65 | 10 |
| 79 | Anipamil | A | Cellulose | 32/61,2/6,8 | 55 | 60 | 70 | 80 | 65 | 65 | 60 | 10 |
| 80 | Anipamil | A | Lactose | 32/34,4/13,6 | 55 | 60 | 70 | 80 | 65 | 65 | 60 | 10 |
| 81 | Anipamil | A | Stärke | 32/54,4/13,6 | 55 | 60 | 70 | 80 | 65 | 65 | 60 | 15 |
| 82 | Coffein Pulver | A | StA | 50/45/5 | 65 | 75 | 90 | 90 | 90 | 90 | 100 | 18 |
| 83 | Coffein Pulver | A | – | 50/50/0 | 65 | 75 | 90 | 90 | 90 | 90 | 100 | 18 |
| 84 | Coffein Granulat | A | StA | 50/45/5 | 65 | 70 | 70 | 75 | 75 | 90 | 80 | 20 |
| 85 | Coffein Granulat | A | – | 50/50/0 | 65 | 70 | 70 | 75 | 75 | 90 | 80 | 20 |

A = Copolymer aus 60 Gew.% NVP und 40 Gew.% Vinylacetat, K-Wert ca. 33
B = PVP, K-Wert 12
C = PVP, K-Wert 17
D = Mowiol® 30-92 (zu 92 % hydrolys. Polyvinylalkohol)
E = Mowiol® 4-80 (zu 80 % hydrolys. Polyvinylalkohol)
F = Copolymer aus NVP, Vinylacetat und Hydroxypropylacrylat im Gewichtsverhältnis 30/40/30;
K-Wert ca. 18
G = Celluloseacetat
H = Celluloseacetat-phthalat
I = Copolymer Vinylacetat/Crotonsäure; K-Wert ca. 30
StA = Stearylalkohol
StS = Stearinsäure
MgSt = Magnesiumstearat

**Patentansprüche**

1. Verfahren zum Tablettieren einer Mischung von mindestens einem pharmazeutischen Wirkstoff und mindestens einem thermoplastischen Polymeren sowie gegebenenfalls üblichen galenischen Hilfsstoffen durch Extrudieren der Mischung und Formgebung, dadurch gekennzeichnet, daß man den noch verformbaren Strang zwischen zwei sich streckenweise auf der Mantelfläche berührenden, gegensinnig umlaufenden und auf der Kontaktstrecke parallel-laufenden Bändern (5) oder zwischen einer Walze (4) und einem auf einem Segment des Walzenmantels aufliegenden und mit diesem umlaufenden Band (5) zu Tabletten verpreßt, wobei die formgebenden, gegebenenfalls sich paarweise ergänzenden Vertiefungen (6) in beiden oder nur in einem der umlaufenden formgebenden Elemente angebracht sind.

2. Verfahren nach Anspruch 1, dadurch gekennzeichnet, daß man zwei elastische Bänder (5) mit einander gegenüberliegenden Vertiefungen (6), die paarweise die Tablettenform bestimmen, einsetzt.

3. Verfahren nach Anspruch 1, dadurch gekennzeichnet, daß man zwei Gliederbänder (5) aus Metall einsetzt, die die paarweise korrespondierenden, formgegebenden Vertiefungen (6) enthalten.

4. Verfahren nach Anspruch 1, dadurch gekennzeichnet, daß das umlaufende, glatte Band (5) durch eine feststehende, in Anpassung an die Walze konkav gewölbte, glatte Wand (7) ersetzt ist.

5. Verfahren nach Anspruch 2, dadurch gekennzeichnet, daß man anstelle des 2. Bandes (5) eine mit dem 1. Band in Kontakt synchron rotierende Walze (4) einsetzt, auf deren Mantel eingravierte formgebende Vertiefungen (6) mit denen des Bandes paarweise korrespondieren.

6. Verfahren nach Anspruch 1, dadurch gekennzeichnet, daß man als thermoplastisches Polymeres ein lösungsmittelfreies N-Vinylpyrrolidon-Polymerisat mit einem Wassergehalt von höchstens 3,5 Gew.% einsetzt, das mindestens 20 Gew.% N-Vinylpyrrolid-2-on (NVP) einpolymerisiert enthält, wobei sämtliche gegebenenfalls einpolymerisierten Comonomeren Stickstoff und/oder Sauerstoff enthalten.

7. Verfahren nach Anspruch 6, dadurch gekennzeichnet, daß man als thermoplastisches Polymeres ein NVP-Polymerisat einsetzt, das entweder in einem organischen Lösungsmittel oder mit einem organischen Peroxid in Wasser hergestellt wurde.

8. Verfahren nach Anspruch 1, dadurch gekennzeichnet, daß man dazu mindestens einen Wirkstoff aus folgender Gruppe einsetzt: Acetaminophen (= Paracetamol), Acetohexamid, Acetyldigoxin, Acetylsalicylsäure, Acromycin, Anipamil, Benzocain, $\beta$-Carotin, Chloramphenicol, Chlordiazepoxid, Chlormadinonacetat, Chlorothiazid, Cinnarizin, Clonazepam, Codein, Dexamethason, Diazepam, Dicumarol, Digitoxin, Digoxin, Dihydroergotamin, Drotaverin, Flunitrazepam, Furosemid, Gramicidin, Griseofulvin, Hexobarbital, Hydrochlorothiazid, Hydrocortison, Hydroflumethiazid, Indomethazin, Ketoprofen, Lonetil, Medazepam, Mefrusid, Methandrostenolon, Methylprednisolon, Methylsulfadiazin (= Sulfaperin), Nalidixinsäure, Nifedipin, Nitrazepam, Nitrofurantoin, Nystatin, Östradiol, Papaverin, Phenacetin, Phenobarbital, Phenylbutazon, Phenytoin, Prednison, Reserpin, Spironolacton, Streptomycin, Sulfadimidin (= Sulfamethazin), Sulfamethizol, Sulfamethoxazol, Sulfamethoxydiazin (= Sulfameter), Sulfaperin, Sulfathiazol, Sulfisoxazol, Testosteron, Tolazamid, Tolbutamid, Trimethoprim, Tyrothricin.

9. Vorrichtung zum Tablettieren einer Mischung von mindestens einem pharmazeutischen Wirkstoff und mindestens einem thermoplastischen Polymeren sowie gegebenenfalls üblichen galenischen Hilfsstoffen durch Extrudieren der Mischung und Formgebung, bestehend aus einem Mischextruder (1) mit einem oder mehreren Einfüllstutzen (2) und mit einer oder mehreren Düsen (3), gekennzeichnet durch zwei anschließend an die Düsen angeordnete, über zwei gegenläufige Walzenpaare (4) angetriebene, in Materialflußrichtung hinter der Extruderdüse zusammenlaufende elastische Bänder (5) aus einem gefüllten Elastomeren, wobei der Füllstoff die Wärmeleitfähigkeit verbessert, mit pro Düse einer oder mehreren, auf jedem Band umlaufenden Reihen von einander gegenüberliegenden Vertiefungen (6), die bei der Berührung der Bänder paarweise die Tablettenform bilden.

10. Vorrichtung zum Tablettieren einer Mischung von mindestens einem pharmazeutischen Wirkstoff und mindestens einem thermoplastischen Polymeren sowie gegebenenfalls üblichen galenischen Hilfsstoffen durch Extrudieren der Mischung und Formgebung, bestehend aus einem Mischextruder (1) mit einem oder mehreren Einfüllstutzen (2) und mit einer oder mehreren Düsen (3), gekennzeichnet durch zwei anschließend an die Düsen angeordnete, über zwei gegenläufige Walzenpaare angetriebene, in Materialflußrichtung hinter der Extruderdüse zusammenlaufende Metallgliederbänder (5) mit pro Düse einer oder mehreren, auf jedem Band umlaufenden Reihen von einander gegenüberliegenden Vertie-

12

fungen (6), die bei der Berührung der Metallglieder paarweise die Tablettenform bilden.

**Claims**

1. A process for tableting a mixture of one or more active pharmaceutical compounds and one or more thermoplastic polymers and, if required, conventional pharmaceutical auxiliaries by extruding the mixture and shaping, wherein the still formable extrudate is pressed to give tablets, between two belts (5) which make contact in parts along their outer surfaces, rotate in opposite directions and run parallel along the contact zone, or between a roller (4) and a belt (5) which rests against a segment of the lateral surface of the roller and revolves with the said surface, the shape-imparting indentations (6), which may be present in complementary pairs, being located in both or in only one of the revolving shape-imparting elements.

2. A process as claimed in claim 1, wherein two resilient belts (5) having indentations (6) which are opposite one another and, in pairs, determine the tablet shape are used.

3. A process as claimed in claim 1, wherein two metal link belts (5) which contain the shape-imparting indentations (6) in corresponding pairs are used.

4. A process as claimed in claim 1, wherein the revolving, smooth belt (5) is replaced by a stationary, smooth wall (7) which is curved in a concave shape to match the roller.

5. A process as claimed in claim 2, wherein, instead of the second belt (5), a roller (4) is used which rotates synchronously in contact with the first belt and on whose lateral surface engraved shape-imparting indentations (6) correspond in pairs with those of the belt.

6. A process as claimed in claim 1, wherein the thermoplastic polymer used is a solvent-free N-vinylpyrrolidone polymer which has a water content of not more than 3.5% by weight and contains not less than 20% by weight of N-vinylpyrrolid-2-one (NVP) as copolymerized units, all comonomers which may be copolymerized containing nitrogen and/or oxygen.

7. A process as claimed in claim 6, wherein the thermoplastic polymer used is an NVP polymer which has been prepared either in an organic solvent or using an organic peroxide in water.

8. A process as claimed in claim 1, wherein one or more active compounds from the following group are used: acetaminophen (= paracetamol), acetohexamide, acetyldigoxin, acetylsalicylic acid, acromycin, anipamil, benzocaine, β-carotene, chloramphenicol, chlordiazepoxide, chlormadinone acetate, chlorothiazide, cinnarizine, clonazepam, codeine, dexamethasone, diazepam, dicumarol, digitoxin, digoxin, dihydroergotamine, drotaverine, flunitrazepam, furosemide, gramicidin, griseofulvin, hexobarbital, hydrochlorothiazide, hydrocortisone, hydroflumethiazide, indomethacin, ketoprofen, lonetil, medazepam, mefruside, methandrostenolone, methylprednisolone, methylsulfadiazine (= sulfaperin), nalidixinic acid, nifedipine, nitrazepam, nitrofurantoin, nystatin, estradiol, papaverine, phenacetin, phenobarbital, phenylbutazone, phenytoin, prednisone, reserpine, spironolactone, streptomycin, sulfadimidine (= sulfamethazine), sulfamethizole, sulfamethoxazole, sulfamethoxydiazine (= sulfameter), sulfaperin, sulfathiazole, sulfisoxazole, testosterone, tolazamide, tolbutamide, trimethoprim and tyrothricin.

9. Apparatus for tableting a mixture of one or more pharmaceutical active compounds and one or more thermoplastic polymers and, if required, conventional pharmaceutical auxiliaries by extruding the mixture and shaping, consisting of a mixing extruder (1) having one or more feed hoppers (2) and having one or more dies (3), wherein, arranged downstream of the dies, there are two resilient belts (5) which are driven via two pairs of rollers (4) rotating in opposite directions, said belts running together in the direction of material flow downstream of the extruder die and consisting of a filler-containing elastomer, the filler improving the thermal conductivity, and having, per nozzle, one or more rows, revolving on each belt, of indentations (6) which are opposite one another and, in pairs, form the tablet shape when the belts come into contact with one another.

10. Apparatus for tableting a mixture of one or more pharmaceutical active compounds and one or more thermoplastic polymers and, if required, conventional pharmaceutical auxiliaries by extruding the mixture and shaping, consisting of a mixing extruder (1) having one or more feed hoppers (2) and having one or more dies (3), wherein, arranged downstream of the dies, there are two metal link belts (5) which are driven via two pairs of rollers rotating in opposite directions, said belts running together in the direction of material flow downstream of the extruder die and having, per nozzle, one or more rows, revolving on each belt, of indentations (6) which are opposite one another and, in pairs, form the tablet shape when the metal links come into contact with one another.

**Revendications**

1. Procédé de transformation en comprimés d'un mélange d'au moins un produit pharmaceutiquement actif et d'au moins un polymère thermoplastique, ainsi qu'éventuellement d'excipients galéniques habituels, par extrusion du mélange et moulage, caractérisé en ce que l'on comprime en comprimés le boudin encore déformable, entre deux bandes (5) dont les surfaces d'enveloppe sont en contact mutuel sur un tronçon étendu, tournant en sens inverse et défilant en parallèle sur l'étendue de leur contact, ou entre un cylindre (4) et une bande (5) reposant sur un segment de l'enveloppe du cylindre et défilant avec celui-ci, les évidements (6) de moulage, qui se complètent éventuellement deux à deux, étant réalisés dans les deux éléments défilants de moulage ou sur un seul des éléments défilants de moulage.

2. Procédé selon la revendication 1, caractérisé en ce que l'on utilise deux bandes élastiques (5) avec des évidements (6) se faisant mutuellement face, qui forment deux à deux le moule à comprimés.

3. Procédé selon la revendication 1, caractérisé en ce que l'on utilise deux bandes (5) à éléments métalliques, qui contiennent les évidements (6) de moulage se correspondant deux à deux.

4. Procédé selon la revendication 1, caractérisé en ce que la bande lisse (5) défilante est remplacée par une paroi lisse (7) cintrée avec une concavité adaptée au cylindre.

5. Procédé selon la revendication 2, caractérisé en ce qu'au lieu de la 2ème bande (5), on utilise un cylindre (4) en contact avec la 1ère bande et tournant en synchronisme avec elle, des évidements (6) de moulage gravés dans son enveloppe correspondant deux à deux à ceux de la bande.

6. Procédé selon la revendication 1, caractérisé en ce que l'on utilise comme polymère thermoplastique un polymère de N-vinylpyrrolidone sans solvant, avec une teneur en eau d'au plus 3,5% en poids, contenant au moins 20% en poids de N-vinylpyrrolidone (NVP) en incorporation polymérique, l'ensemble des comonomères éventuellement incorporés par polymérisation avec lui contenant de l'azote et/ou de l'oxygène.

7. Procédé selon la revendication 6, caractérisé en ce que l'on utilise comme polymère thermoplastique un polymère de NVP, qui a été produit dans un solvant organique, ou bien dans l'eau avec un peroxyde organique.

8. Procédé selon la revendication 1, caractérisé en ce que l'on y utilise au moins un produit actif du groupe suivant : acétaminophène (= paracétamol), acétohexamide, acétyldigoxine, acide acétylsalicylique, acromycine, anipamil, benzocaïne, $\beta$-carotine, chloramphénicol, chlordiazépoxide, acétate de chlormadinone, chlorothiazide, cinnarizine, clonazépam, codéine, dexaméthasone, diazépam, dicumarol, digitoxine, digoxine, dihydroergotamine, drotavérine, flunitrazépam, furosémide, gramicidine, griséofulvine, hexobarbital, hydrochlorothiazide, hydrocortisone, hydrofluméthiazide, indométhazine, cétoprofène, lonétil, médazépam, méfruside, méthandrosténolone, méthylprednisolone, méthylsulfadiazine (= sulfapérine), acide nalidixinique, nifédipine, nitrazépam, nitrofurantoïne, nystatine, oestradiol, papavérine, phénacétine, phénobarbital, phénylbutazone, phénytoïne, prednisone, réserpine, pironolactone, streptomycine, sulfadimidine (= sulfaméthazine), sulfaméthizol, sulfaméthoxazol, sulfaméthoxydiazine (= sulfaméter), sulfapérine, sulfathiazol, sulfisoxazol, testostérone, tolazamide, tolbutamide, triméthoprime, tyrothrycine.

9.  Installation de transformation en comprimés d'un mélange d'au moins un produit pharmaceutiquement actif et d'au moins un polymère thermoplastique, avec éventuellement des excipients galéniques habituels, par extrusion du mélange et moulage, constitué d'une mélangeuse extrudeuse (1) avec un ou plusieurs raccords de remplissage (2) et avec une ou plusieurs filières (3), caractérisée par deux bandes (5) élastiques en un élastomère chargé, disposées à la suite des filières, entraînées par l'intermédiaire de deux paires de cylindres (4) tournant en sens inverse, défilant ensemble en arrière des filières de l'extrudeuse dans la direction d'écoulement de la matière, la charge améliorant la conductibilité thermique, avec pour chaque filière, une ou plusieurs séries, disposées dans la direction du défilement de chaque bande, d'évidements (6) se faisant face deux à deux qui, lorsque les bandes sont en contact, forment deux à deux les moules à comprimés.

10. Installation de transformation en comprimés d'un mélange d'au moins un produit pharmaceutiquement actif et d'au moins un polymère thermoplastique, avec éventuellement des excipients galéniques habituels, par extrusion du mélange et moulage, constituée d'une mélangeuse extrudeuse (1) avec un ou plusieurs raccords de remplissage (2) et avec une ou plusieurs filières (3), caractérisée par deux bandes (5) à éléments métalliques, disposées à la suite des filières, entraînées par l'intermédiaire de deux paires de cylindres (4) tournant en sens inverse, défilant ensemble en arrière des filières de l'extrudeuse, dans la direction d'écoulement de la matière, avec pour chaque filière une ou plusieurs séries, disposées dans la direction du défilement de chaque bande, d'évidements (6) se faisant face deux à deux qui, lorsque les bandes sont en contact, forment deux à deux les moules à comprimés.

FIG.1

4   6   5

2

1   3

4   6   5

FIG.2

4

6   5

2   3

1

4

FIG.3

FIG.4

FIG. 5